# EUROPEAN PATENT SPECIFICATION

(11) **EP 4 413 194 B1**
(45) Date of publication and mention of the grant of the patent: **25.06.2025**
(21) Application number: 22789319.5
(22) Date of filing: 06.10.2022
(51) Int. Cl.: D04H 1/495, D04H 1/498, D04H 1/26, D04H 1/425, D04H 1/4258, A61K 8/02, A61Q 1/14, A61Q 15/00, A61Q 17/00, A61Q 19/00, A61Q 19/10

(54) **BIODEGRADABLE WIPE**
BIOLOGISCH ABBAUBARES WISCHTUCH
LINGETTE BIODÉGRADABLE

(30) Priority: 06.10.2021 DK PA202170495; 06.10.2021 US 202163252649 P
(43) Date of publication of application: 14.08.2024
(73) Proprietor: Glatfelter Holding (Switzerland) AG, 4052 Basel (CH)
(72) Inventor: SINGH, Vinitkumar, Nashville, Tennessee 37214 (US)
(74) Representative: Chas. Hude A/S
(86) International application number: PCT/IB2022/059545
(87) International publication number: WO 2023/057945

(56) References cited:
- EP-A1- 3 715 514
- EP-A2- 1 310 226
- WO-A1-2013/047862
- WO-A1-2018/193775
- WO-A2-03/016445
- US-A1- 2004 255 440
- US-A1- 2006 063 456

## Description

### FIELD OF THE INVENTION

The present invention relates to a biodegradable wipe having a three-dimensional perforated structure pattern effective for trapping and removing oily and sticky liquids without scratching underlying surfaces or irritating skin, further said wipe is biodegradable, home compostable and shows duality by providing a scrubbing effect when dry and smoothing or gentle effect when wet. Further, the wipe has personal care and skin care applications.

### BACKGROUND OF THE INVENTION

A large amount of oil is constantly secreted from the face, especially from the skin of the nose, cheeks and forehead. In order to maintain cleanliness and improve the malleability of cosmetics, it is essential to remove any excess oil or sebum and so, for removing such oil on the face a wipe material is commonly used. Conventional paper wipes have been used to remove facial oils. For example, natural or synthetic papers using vegetable fibres, synthetic pulp have been used. However, these paper wipes often irritate the skin due to the rigid and stiff nature of the fibres. In order to improve the smoothness of the blotter paper, these papers have undergone calendering and / or coating treatment with powders such as calcium carbonate and sizing agents. However, calendering treatment can deform to a rough surface when a significant amount of binder or sizing agent is not used, and oil absorption is reduced. In addition, the paper wipe is a poor indicator in terms of its efficiency since the paper does not change significantly when the paper absorbs oil or sebum.

Also, there are many disposable wipes that comprise a single layer having a single textured surface and cannot provide both gentle and vigorous cleansing from the same wipe. Moreover, many single layered wipes lack the rigidity and integrity required for effective cleansing and/or treatment. The rigidity and integrity of these wipes has been improved by increasing their thickness or by laminating them to a second nonwoven layer to form a dual layered nonwoven wipe. However, these dual layered wipes are not very cost effective for onetime use. Also known are dual layered pads which comprise a paper pad layer laminated to a high-loft nonwoven pad layer. However, these dual layered pads are still relatively expensive for one time use and often irritate the skin causing scratches on the surface of the skin. Therefore, it would be highly desirable to develop a biodegradable wipe using hydraulic entanglement of fibres having an aperture, textured, repeating pattern on the surface which provides sufficient rigidity and integrity for removing dirt and oil from the underlying surface without causing scratches on the underlying surface or irritating skin, is useful for both gentle and vigorous cleansing depending upon the condition of the wipe and is cost effective in nature.

The pattern of the wipe provides a macroscopic texture that is smoother and less irritating to the skin than more expensive high-loft nonwovens and is effective for trapping and removing oily and sticky liquids without scratching underlying surfaces or irritating skin. The dual nature of the wipe in the present invention allows it to be used, for example, for scrubbing when in dry condition and for gentle cleansing when in wet condition. Furthermore, the patterned or aperture on the surface of the wipe tend to concentrate removed soil and dirt on the raised areas, thereby enhancing the user's perception of cleansing efficacy. The biodegradable wipes of the present invention are useful for delivering a wide variety of cleansing compositions, pharmaceutical actives, and cosmetics, including astringents, toners, lotions, emulsions, moisturizers, and the like. These wipes are especially well suited for delivering cleansers and compositions for treating skin. These wipes are also useful for delivering compositions for the regulation of skin wrinkles and/or atrophy. Also, these wipes are useful for make-up removal.

It is therefore an object of the present invention to provide a biodegradable wipe with a three dimensional perforated structure pattern having improved efficacy and aesthetics, and yet which are also cost effective and durable. Another object of the present invention is to provide a three dimensional perforated structure on a wipe effective for trapping and removing oily and sticky liquids without scratching underlying surfaces or irritating skin. Yet another object of the present invention is to provide a biodegradable wipe provides a scrubbing effect when dry and smoothing or gentle effect when wet.

WO2013/047862 discloses a dual-sided wet wipe with different 3D groove patterns on opposite sides, with one side being for coarse removal of soil and the other side for a fine, finishing removal of any soil remaining after coarse cleaning. The wipe comprises a spunlaced web of softwood pulp and rayon fibres.

These and other objects of this invention will become apparent in light of the following disclosure.

### SUMMARY OF THE INVENTION

The present invention relates to a biodegradable wipe having a three-dimensional perforated structure pattern effective for trapping and removing oily and sticky liquids without scratching underlying surfaces or irritating skin, as defined in claim 1. Certain embodiments of the wipe are defined in dependent claims 2-6. The wipe is biodegradable and shows duality by providing a scrubbing effect when dry and smoothing or gentle effect when wet.

The present invention provides a biodegradable wipe having a three-dimensional perforated structure pattern comprising: a spunlaced unitary structure of a randomised web of spunlaced cellulosic fibres and a sheet of wood pulp fibres; a three dimensional perforated structure pattern in said unitary structure; and optionally a colorant; wherein said wipe provides a scrubbing effect when dry and smoothing or gentle effect when wet; and said three dimensional perforated structure is effective for trapping and removing oily and sticky liquids without scratching underlying surfaces or irritating skin. The wipe is biodegradable, home compostable and dual in nature and has personal care and skin care applications.

In another embodiment, the present invention provides a three dimensional perforated structure pattern on a wipe, wherein said three dimensional perforated structure is effective for trapping and removing oily and sticky liquids without scratching underlying surfaces or irritating skin.

### DETAILED DESCRIPTION OF THE INVENTION

The following description provides a general description of the present invention and specific preferred embodiments. However, one of ordinary skill will recognize that many variations of the invention may be possible without departing from the gist of the invention.

This description and the following claims are intended to include all such variation. In a preferred embodiment, the present invention provides a three dimensional perforated structure pattern on a wipe, wherein said three dimensional perforated structure is effective for trapping and removing oily and sticky liquids without scratching underlying surfaces or irritating skin. The said wipe shows duality by providing a scrubbing effect when dry and smoothing effect when wet.

In another preferred embodiment, the present invention provides a biodegradable wipe having a three-dimensional perforated structure pattern comprising: a spunlaced unitary structure of a randomised web of spunlaced cellulosic fibres and a sheet of wood pulp fibres in a desired proportion; a three dimensional perforated structure pattern on a surface of said wipe; and optionally a colorant; wherein said wipe provides a scrubbing effect when dry and smoothing or gentle effect when wet; and said three dimensional perforated structure is effective for trapping and removing oily and sticky liquids without scratching underlying surfaces or irritating skin. The wipe is biodegradable, home compostable and dual in nature and has personal care and skin care applications.

The wipe has opposing surfaces wherein said opposing surfaces having one side selected from a group of fibres which includes but not limited to wood pulp and other side selected from a group of fibres which includes but not limited to lyocell, rayon, raw and bleached cotton fibres. The combined web/sheet structure is further spunlaced to form a unitary sheet structure having opposing surfaces that are wood pulp-rich and cellulose-rich. The softwood pulp fibre was fed in sheet form to a position atop the lyocell fibre web. The unitary sheet structure may be described in terms of forming a web having a plurality of fibres disposed in a generally random pattern throughout the fibrous structure. A plurality of natural fibres may also be disposed in a generally random pattern throughout the fibrous structure. In another embodiment, a portion of the fibres may be redistributed in a non-random repeating pattern. Layered deposition of the fibres, synthetic and natural, is also contemplated by the present invention. The resulting fibrous structure may comprise natural and synthetic fibres dispersed generally randomly throughout the layer. Alternatively, the natural and synthetic fibres may be more structured such that the synthetic fibres and natural fibres may be disposed generally non-randomly.

As used herein, "perforated structure pattern" means any screen, apertured, perforated or grooved plate, or the like, on which the hydroentangled web of fibres is supported during processing and which by reason of its apertures and/or surface contours influences the movement of the fibres into a pattern in response to water jet streams. The patterning member may have a planar or nonplanar surface or a combination of planar and nonplanar areas. Various weaves and patterns may also be selected for the apertured patterning member according to the fabric pattern desired. The pattern is determined by the pattern of openings in the perforated structure pattern of said wipe. As a result, patterning is a critical element in producing wipes or fabric that will satisfy the requirements necessary for trapping and removing oily and sticky liquids without scratching underlying surfaces or irritating skin or other cosmetic applications. The perforated structure on said wipe in the present invention is useful as body wipes, baby wipes, face wipes, make up removing wipes and other end-use applications where trapping and removing oily and sticky liquids without scratching underlying surfaces or irritating skin is important.

The pattern of the wipe provides a macroscopic texture that is smoother and less irritating to the skin than more expensive high-loft nonwovens and is effective for trapping and removing oily and sticky liquids without scratching underlying surfaces or irritating skin.

The dual nature of the wipe in the present invention allows it to be used, for example, for scrubbing when in dry condition and for gentle cleansing when in wet condition. Furthermore, the patterned or aperture on the surface of the wipe tend to concentrate removed soil and dirt on the raised areas, thereby enhancing the user's perception of cleansing efficacy. The biodegradable wipes of the present invention are useful for delivering a wide variety of cleansing compositions, pharmaceutical actives, and cosmetics, including astringents, toners, lotions, emulsions, moisturizers, and the like. These wipes are especially well suited for delivering cleansers and compositions for treating skin. These wipes are also useful for delivering compositions for the regulation of skin wrinkles and/or atrophy. Also, these wipes are useful for make-up removal.

In yet another preferred embodiment, said biodegradable wipe further comprises at least one component selected from the group comprising of a cleansing agent, a sterilizing agent, a deodorizing agent, a disinfectant, a moisturizing agent and a cosmetic remover.

The present invention provides a biodegradable wipe having a three-dimensional perforated structure pattern comprising: spunlaced unitary structure of a randomised web of spunlaced cellulosic fibres and a sheet of wood pulp fibres in a desired proportion; a three dimensional perforated structure pattern on said wipe; and optionally a colorant; wherein said wipe provides a scrubbing effect when dry and smoothing effect when wet; said cellulosic fibres selected from group of fibres which include but are not limited to man-made cellulosic fibres, natural cellulose fibres, regenerated cellulosic fibres, synthetic fibres and protein fibres or mixtures thereof and said three dimensional perforated structure is effective for trapping and removing oily and sticky liquids without scratching underlying surfaces or irritating skin. Further, the biodegradable wipe comprises pulp fibres which are wood pulp fibres from conventional sources. Such fibres may be chemically pulped, unbleached or bleached, virgin or recycled, hardwood or softwood fibres.

According to the present invention the spunlaced cellulose fibres are about 25% to about 75% wood pulp fibres and about 25% to about 75% fibres which include but are not limited to man-made cellulosic fibres, natural cellulose fibres, regenerated cellulosic fibres, synthetic fibres and protein fibres or mixtures thereof. Exemplary of such fibres include but are not limited to natural fibres such as wool, silk, jute, hemp, cotton, linen, sisal, or ramie and the synthetic or man-made cellulosic fibres, notably rayon or regenerated cellulose, lyocell fibres, viscose fibres, cellulose acetate fibres, and mixtures thereof. Other fibres of a synthetic or man-made origin may be selected from group but not limited to: polyamide fibres such as nylon 6, nylon 66, nylon 610, etc.; polyester fibres such as "Dacron", "Fortrel" and "Kodel"; acrylic fibres such as "Acrilan", "Orlon" and "Creslan"; modacrylic fibres derived from polyethylene and polypropylene; cellulose ester fibres such as "Arnel" and "Acele"; polyvinyl alcohol fibres, etc.

More particularly, the randomised web of spunlaced cellulose fibres are about 55% soft pulp fibres and 45% fibres include but are not limited to which include but are not limited to man-made cellulosic fibres, natural cellulose fibres, regenerated cellulosic fibres, synthetic fibres and protein fibres or mixtures thereof. The performance properties of the hydroentangled web may be modified by variation of the content of the natural cellulose fibres and one of skill in the art will recognize that the composition content within the described ranges may be varied depending on the source and history of the cellulose fibres. The wood pulp fibres are low cost, soft and easily colored. The regenerated cellulosic fibres of lyocell or rayon provide a suitable strength level. The strength of the fabric also arises from the entanglement of the fibres and thus precludes the need to add fibre binding materials such as adhesives or conjugate binder fibres.

In producing the fabric, the fibres are made into a web and typically consolidated by spunlacing. Wood pulp fibre in sheet form which may be colored due to presence of lignins in wood pulp is then positioned onto the surface of the web of the fibres and is usually darker in color than the cellulose-rich surface. The combined web/sheet structure is further spunlaced to form a substantially unitary sheet structure having opposing surfaces that are wood pulp-rich and desired fibre-rich which includes but not limited to man-made cellulosic fibres, natural cellulose fibres, regenerated cellulosic fibres, synthetic fibres and protein fibres or mixtures thereof. The opposing surfaces of the wipe having one side selected from a group of fibres which includes but not limited to wood pulp and other side selected from a group of fibres which includes but not limited to man-made cellulosic fibres, natural cellulose fibres, regenerated cellulosic fibres, synthetic fibres and protein fibres or mixtures thereof. The biodegradable fabric may or may not be colored, wherein the color is produced during spunlacing step.

Optionally, a colorant such as a dye or pigment can be added. The dye or pigment may also use a fixative or adhesive material to attach it to the cellulosic fibres, but the fixative or adhesive material does not bond the cellulosic fibres together.

The biodegradable wipe is made in a hydroentanglement process. Hydroentanglement is also known as spunlacing. Hydroentanglement is a fabrication technique wherein the individual fibres are entangled using high-pressure water jets. When fabricating a non-woven fabric using hydroentanglement, a plurality of non-entangled individual fibres is arranged in a fibrous web using at least one carding machine followed by moving said fibrous web into a hydroentanglement section, wherein multiple high-pressure water jets are introduced, said water jets penetrating the fibrous web and causing entanglement and thereby (mutual) physical bonding of the textile fibres. Thereby, the entanglement of textile fibres creates a non-woven fabric. The fibrous web may be formed using at least one carding machine, but may also be formed by other means. The movement of the fibrous web between the carding machine and the hydroentanglement section may be carried out by means of a conveyor belt, the speed of said movement partly affecting the orientation of the fibres. In other words, the fibrous web is laid down on a conveyor belt by the carding machine and subsequently transported into the hydroentanglement section.

In yet another embodiment, the biodegradable wipe of the present invention having a three-dimensional perforated structure pattern comprising: a spunlaced unitary structure of a randomised a web of spunlaced cellulosic fibres selected from but not limited to man-made cellulosic fibres, natural cellulose fibres, regenerated cellulosic fibres, synthetic fibres and protein fibres or mixtures thereof in a desired proportion; a three dimensional perforated structure pattern on said wipe; and optionally a colorant; wherein said wipe comprising about 55% softwood pulp fibre and about 45% fibres which include but are not limited to man-made cellulosic fibres, natural cellulose fibres, regenerated cellulosic fibres, synthetic fibres and protein fibres or mixtures thereof, wherein cellulosic fibre is prepared by a process comprising the steps of: generating a web of lyocell or rayon fibre by spunlacing including at least hydraulic entanglement of the fibres, to increase the strength of the web and produce a bonded web of nonwoven fabric, wherein the web is at least 17g/m²; feeding at least one sheet of the softwood pulp atop the web of desired fibre which include but are not limited to man-made cellulosic fibres, natural cellulose fibres, regenerated cellulosic fibres, synthetic fibres and protein fibres or mixtures thereof creating a combined web/ structure; spunlacing the combined web/sheet structure to form the biodegradable wipe with a basis weight in range of 40g/m² to 150g/m²; creating the biodegradable wipe wherein the substantially separated opposing surfaces include a desired fibre side and a softwood pulp fibre side. Said wipe provides a scrubbing effect when dry and smoothing effect when wet; and said three dimensional perforated structure pattern is effective for trapping and removing oily and sticky liquids without scratching underlying surfaces or irritating skin.

### EXAMPLE

The following examples further describe and demonstrate embodiments within the scope of the present invention. The examples are given solely for the purpose of illustration and are not to be construed as limitations of the present invention, as many variations thereof are possible without departing from the scope of the invention.

A biodegradable wipe of the present invention having a three-dimensional perforated structure pattern comprising: a spunlaced unitary structure of a sheet of wood pulp fibres and a randomised a web of cellulosic fibres selected from group comprising of lyocell, rayon, or a combination thereof in a desired proportion; a three dimensional perforated structure pattern on said wipe; and optionally a colorant; wherein said wipe comprising about 55% softwood pulp fibre and about 45% lyocell or rayon regenerated cellulosic fibres, wherein cellulosic fibre is prepared by a process comprising the steps of: generating a web of lyocell or rayon fibre by spunlacing including at least hydraulic entanglement of the fibres, to increase the strength of the web and produce a bonded web of nonwoven fabric, wherein the web is atleast 17 g/m²; feeding at least one sheet of the softwood pulp atop the web of lyocell or rayon creating a combined web/ structure; spunlacing the combined web/sheet structure to form the biodegradable wipe with a basis weight of about 71g/m²; creating the biodegradable wipe wherein the substantially separated opposing surfaces include a spunlaced lyocell or rayon fibre side and a softwood pulp fibre side. Said wipe provides a scrubbing effect when dry and smoothing effect when wet; and said three dimensional perforated structure pattern is effective for trapping and removing oily and sticky liquids without scratching underlying surfaces or irritating skin.

The drape, softness, comfortable hand of the web may be controlled by the energy delivered by the high pressure jets and by the speed of travel of the web through the equipment. Additionally, the porosity and absorbency of the nonwoven web may be affected by these parameters along with the content and structure of the bonding fibres. According to the present invention by control of both water pressure and speed of web travel through the spunlacing equipment, the composition make-up of natural cellulosic fibres, manmade and bonding fibres as well as the absence of adhesives and binders, a nonwoven web having varying degrees of strength, absorbency, softness and thickness may be obtained. Further, the bonded nonwoven web is free on adhesives and binders it is hygienically safe and substantially free of lint and dust particles.

The above description is presented to enable a person skilled in the art to make and use the invention, and is provided in the context of a particular application and its requirements. Various modifications to the preferred embodiments will be readily apparent to those skilled in the art, and the generic principles defined herein may be applied to other embodiments and applications without departing from the scope of the invention. Thus, this invention is not intended to be limited to the embodiments shown, but is to be accorded the widest scope consistent with the principles and features disclosed herein. In this regard, certain embodiments within the invention may not show every benefit of the invention, considered broadly.

## Claims

1. A biodegradable wipe having a three-dimensional perforated structure pattern comprising:
a spunlaced unitary structure of a randomised web of spunlaced cellulosic fibres and a sheet of
wood pulp fibres;
a three dimensional perforated structure pattern in said unitary structure; and
optionally a colorant;
wherein said cellulosic fibres are selected from the group of lyocell, rayon, raw and bleached cotton, or a combination thereof;
wherein said wipe provides a scrubbing effect when dry and a smoothing effect when wet; and
wherein said three dimensional perforated structure is effective for trapping and removing oily and sticky liquids without irritating the skin;
**characterized in that** the spunlaced unitary structure has opposing surfaces, with the wood pulp fibre sheet on one side and the randomized web of cellulosic fibres on the opposite side.

2. The biodegradable wipe of claim 1, wherein the unitary structure has a basis weight in the range of 40g/m² to 150g/m².

3. The biodegradable wipe of claim 1, further comprising at least one component selected from the group comprising of a cleansing agent, a sterilizing agent, a deodorizing agent, a disinfectant, a moisturizing agent and a cosmetic remover.

4. The biodegradable wipe of claim 1, wherein the spunlaced unitary structure fibres are about 25% to about 75% wood pulp fibres and about 25% to about 75% fibres including man-made cellulosic fibres and natural cellulose fibres or mixtures thereof.

5. The biodegradable wipe of claim 1, wherein the opposing surfaces has one side selected from a group of fibres which includes but not limited to wood pulp and other side selected from a group of fibres which includes but not limited to spunlaced regenerated cellulose fibres and natural cellulose fibres.

6. The biodegradable wipe of claim 1, wherein the spunlaced unitary structure fibres are about 55% softwood pulp fibres and 45% fibres including man-made cellulosic fibres and natural cellulose fibres or mixtures thereof.

7. Use of the wipe as claimed in claim 1, for personal care and skin care applications.

## Patentansprüche

1. Biologisch abbaubares Wischtuch mit einem dreidimensionalen perforierten Strukturmuster, umfassend:
eine Spunlace-Einheitsstruktur aus einem randomisierten Gewebe aus Spunlace zellulosischen Fasern und einer Lage aus Zellstofffasern;
ein dreidimensionales perforiertes Strukturmuster in dieser Einheitsstruktur; und gegebenenfalls ein Farbmittel;
wobei die zellulosischen Fasern ausgewählt sind aus der Gruppe von Lyocell, Rayon, roher und gebleichter Baumwolle, oder einer Kombination davon;
wobei das Wischtuch im trockenen Zustand eine Reibungswirkung und im nassen Zustand eine Glättungswirkung aufweist; und
wobei die dreidimensionale perforierte Struktur wirksam ist, um ölige und klebrige Flüssigkeiten aufzufangen und zu entfernen, ohne die Haut zu reizen;
**dadurch gekennzeichnet, dass** die Spunlace-Einheitsstruktur gegenüberliegende Oberflächen aufweist, wobei sich die Zellstofffaserschicht auf einer Seite und das randomisierte Gewebe aus zellulosischen Fasern auf der gegenüberliegenden Seite befindet.

2. Biologisch abbaubares Wischtuch nach Anspruch 1, wobei die Einheitsstruktur ein Flächengewicht im Bereich von 40 g/m² bis 150 g/m² hat.

3. Biologisch abbaubares Wischtuch nach Anspruch 1, ferner umfassend mindestens eine Komponente, die ausgewählt ist aus der Gruppe umfassend ein Reinigungsmittel, ein Sterilisierungsmittel, ein Desodorierungsmittel, ein Desinfektionsmittel, ein Feuchtigkeitsmittel und einen kosmetischen Entferner.

4. Biologisch abbaubares Wischtuch nach Anspruch 1, wobei die Spunlace Einheitssturkurfasern zu etwa 25 % bis etwa 75 % aus Zellstofffasern und zu etwa 25 % bis etwa 75 % aus Fasern bestehen, die künstliche zellulosische Fasern und natürliche Zellulosefasern oder Mischungen davon umfassen.

5. Biologisch abbaubares Wischtuch nach Anspruch 1, wobei die gegenüberliegenden Oberflächen eine Seite aufweisen, die aus einer Gruppe von Fasern ausgewählt ist, die Zellstoff einschließt, aber nicht darauf beschränkt ist, und eine andere Seite, die aus einer Gruppe von Fasern ausgewählt ist, die Spunlace regenerierte Zellulosefasern und natürliche Zellulosefasern einschließt, aber nicht darauf beschränkt ist.

6. Biologisch abbaubares Wischtuch nach Anspruch 1, wobei die Spunlace-Einheisstrukturfasern zu etwa 55 % aus Weichholz-Zellstofffasern und zu 45 % aus Fasern bestehen, die künstliche zellulosische Fasern und natürliche Zellulosefasern oder Mischungen davon einschließen.

7. Verwendung des Wischtuchs nach Anspruch 1 für Körperpflege- und Hautpflegeanwendungen.

## Revendications

1. Lingette biodégradable avec un motif de structure perforée tridimensionnelle comprenant :
une structure unitaire non-tissée spunlace d'une toile aléatoire de fibres cellulosiques non-tissées spunlace et une nappe de fibres de pâte de bois ;
un motif de structure perforée tridimensionnelle dans ladite structure unitaire ; et
éventuellement un colorant ;
dans laquelle lesdites fibres cellulosiques sont choisies dans le groupe constitué de lyocell, de rayonne, de coton brut et blanchi, ou une combinaison de ceux-ci ;
dans laquelle ladite lingette a un effet de frottement à l'état sec et un effet de lissage à l'état humide ; et
dans laquelle ladite structure perforée tridimensionnelle est efficace pour piéger et enlever des liquides huileux et collants sans irriter la peau ;
**caractérisée en ce que** la structure unitaire non-tissée spunlace comprend des surfaces opposées, avec la nappe de fibres de pâte de bois sur un côté et la toile aléatoire de fibres cellulosiques sur le côté opposé.

2. Lingette biodégradable selon la revendication 1, dans laquelle la structure unitaire présente un poids de base compris entre 40g/m² et 150 g/m².

3. Lingette biodégradable selon la revendication 1, comprend en outre au moins un composant choisi dans le groupe comprenant un agent nettoyant, un agent stérilisant, un agent désodorisant, un désinfectant, un agent hydratant et un démaquillant cosmétique.

4. Lingette biodégradable selon la revendication 1, dans laquelle les fibres avec une structure unitaire non-tissée spunlace sont constituées d'environ 25 % à environ 75 % de fibres de pâte de bois et d'environ 25 % à environ 75 % de fibres comprenant des fibres cellulosiques synthétiques et des fibres cellulosiques naturelles ou des mélanges de celles-ci.

5. Lingette biodégradable selon la revendication 1, dans laquelle les surfaces opposées comprennent un côté choisi dans un groupe constitué par des fibres qui comprennent, mais sans s'y limiter, la pâte de bois et un autre côté choisi dans un groupe constitué par des fibres qui comprennent, mais sans s'y limiter, des fibres cellulosiques régénérées non-tissées spunlace et des fibres de cellulose naturelles.

6. Lingette biodégradable selon la revendication 1, dans laquelle les fibres avec une structure unitaire non-tissée spunlace sont constituées d'environ 55 % de fibres de pâte de bois et 45 % de fibres constituées par des fibres cellulosiques synthétiques et des fibres cellulosiques naturelles ou des mélanges de celles-ci.

7. Utilisation de la lingette selon la revendication 1, pour les applications de soins personnels et de soins de peau.
